# EUROPÄISCHE PATENTSCHRIFT

(11) **EP 0 745 593 B1**
(45) Veröffentlichungstag und Bekanntmachung des Hinweises auf die Patenterteilung: **17.01.2001**
(21) Anmeldenummer: 96108151.0
(22) Anmeldetag: 22.05.1996
(51) Int. Cl.: C07D 239/30

(54) **Verfahren zur Herstellung von reinem 4,6-Dichlorpyrimidin**
Process of preparation of pure 4,6-dichloropyrimidine
Procédé pour la préparation de 4,6-dichlorompyrimidine pure

(30) Priorität: 02.06.1995 AT 93995
(43) Veröffentlichungstag der Anmeldung: 04.12.1996
(73) Patentinhaber: DSM Fine Chemicals Austria GmbH, 4021 Linz (AT)
(72) Erfinder: Huber, Wolfgang, Dr., 5020 Salzburg (AT); Schwarz, Franz-Thomas, Dr., 4493 Wolfern (AT); Heu, Ferdinand, 4030 Linz (AT)
(74) Vertreter: Klostermann, Ingrid

(56) Entgegenhaltungen:
- EP-A- 0 329 170
- WO-A-91/01310
- WO-A-95/07265
- WO-A-95/29166
- J. CHEM. SOC., 1951, LONDON, Seite 2214 XP000577048 R. HULL: "A new synthesis of 4:6-Dihydroxypyrimidines"

## Beschreibung

4,6-Dichlorpyrimidin (DCP) ist ein bekanntes und wertvolles Zwischenprodukt für Medikamente und Agrarchemikalien. Aus der Literatur sind daher bereits einige Verfahren zur Herstellung von DCP bekannt.

So ist beispielsweise in J. Chem. Soc (1943) S. 575, und J. Chem. Soc. (1951) S. 2214 ein Verfahren beschrieben, bei dem 4,6-Dihydroxypyrimidin (DHP) mit einem Überschuß an Phosphoroxychlorid und Dimethylanilin als Säurefänger unter Rückflußkochen zu DCP umgesetzt wird. Die Isolierung von DCP erfolgt dabei nach Abdestillieren von überschüssigem Phosphoroxychlorid durch Gießen auf Eiswasser mit anschließender Extraktion mittels Ether. Der so erhaltene Extrakt wird dann mit einer Natriumcarbonatlösung gewaschen, getrocknet und eingedampft. Die Ausbeuten bei diesen Verfahren liegen bei 75,2 % bzw. nach anschließender Destillation oder Rekristallisation bei 51,3 %. Bei diesem Verfahren entstehen jedoch bei der Aufarbeitung durch das Gießen auf Eiswasser große Mengen des Hydrolyseproduktes 4-Chlor-6-hydroxypyrimidin, wodurch sowohl die Ausbeute an DCP als auch dessen Reinheit stark beeinträchtigt werden und ein zusätzlicher Reinigungsschritt erforderlich wird. Ein weiteres Problem bei diesem Verfahren ist die Art der Trocknung von DCP, da dieses auf Grund seines hohen Dampfdruckes leicht sublimiert.

Aus EP-A-329 170 ist ein Verfahren zur Herstellung von 2-Amino-4,6-dichlorpyrimidin (ADCP) durch Chlorierung der entsprechenden Dihydroxyverbindung mit POCl₃ in Gegenwart eines Säurefängers und in Anwesenheit eines zusätzlichen Lösungsmittels bekannt.
Als geeignete Säurefänger werden Trialkylamine, Dialkylamine, Dialkylaniline, Pyridine u.s.w. genannt. Zur Isolierung von ADCP wird das Reaktionsgemisch mit Eiswasser vermischt und anschließend wieder erwärmt.

WO-A-9507265 beschreibt ebenfalls ein Verfahren zur Herstellung von ADCP, jedoch ohne zusätzlichem Lösungsmittel. Als Säurefänger werden Amine, unter anderem Triethylamin, bevorzugt jedoch N,N-Dimethylamin eingesetzt. Die Isolierung erfolgt durch Quenchen.

Aufgabe der vorliegenden Erfindung war es demnach, ein einfaches Verfahren zur Herstellung von reinem 4,6-Dichlorpyrimidin zu finden.
Unerwarteterweise konnte diese Aufgabe durch das erfindungsgemäße Verfahren gelöst werden.

Gegenstand der vorliegenden Erfindung ist demnach ein Verfahren zur Herstellung von reinem 4,6-Dichlorpyrimidin durch Reaktion von 4,6-Dihydroxypyrimidin mit einem Überschuß an Phosphoroxychlorid und in Gegenwart eines Säurefängers und Katalysators, das dadurch gekennzeichnet ist, daß ein Trialkylamin mit 2 bis 4 C-Atomen im Alkylteil als Säurefänger und Katalysator verwendet wird, und daß zur Isolierung von 4,6-Dichlorpyrimidin überschüssiges Phosphoroxychlorid abdestilliert und der Rückstand, sowie ein Neutralisierungsmittel in Wasser bei 30 bis 70°C eindosiert wird, sodaß ein pH-Wert zwischen 2 und 5 erreicht wird, worauf nach 10 bis 60 minütigem Rühren und anschließendem Abkühlen auf 10 bis 30°C 4,6-Dichlorpyrimidin ausfällt.

Bei dem erfindungsgemäßen Verfahren wird 4,6-Dihydroxypyrimidin mit einem Überschuß an POCl₃ zu 4,6-Dichlorpyrimidin umgesetzt. DHP wird dazu in POCl₃ suspendiert, das sowohl als Reagens als auch als Reaktionsmedium dient. Durch die eingesetzte Menge an POCl₃ wird die Handhabbarkeit der Suspension bestimmt. Das molare Verhältnis von DHP zu POCl₃ kann dabei variieren, wobei die Untergrenze durch die Rührbarkeit der Suspension und die Obergrenze hauptsächlich durch wirtschaftliche Faktoren beeinflußt wird. Bevorzugt wird ein molares Verhältnis von DHP zu POCl₃ von etwa 1 : 3 bis 1 : 8, besonders bevorzugt von etwa 1 : 4 bis 1 : 6 eingesetzt. Ein größerer Überschuß an POCl₃ hat keine nachteiligen Auswirkungen auf die Reaktion und kann gewünschtenfalls auch eingesetzt werden, ist jedoch aus wirtschaftlichen Gründen meist nicht sinnvoll. Zu dieser Suspension wird ein Trialkylamin mit 2 bis 4 C-Atomen im Alkylteil zudosiert. Geeignete Trialkylamine sind dabei Triethylamin, Tri-n-butylamin und Tripropylamin. Bevorzugt wird Triethylamin eingesetzt. Die Zudosierung erfolgt bevorzugt über einen Zeitraum von etwa 20 bis 240 Minuten, besonders bevorzugt von 60 bis 150 Minuten, wodurch die Temperatur je nach Dosiergeschwindigkeit und Verdünnung bis zur Rückflußtemperatur ansteigen kann, bevorzugt wird jedoch so schnell zudosiert, daß die Temperatur nicht über 90°C ansteigt. Die Menge an zudosiertem Trialkylamin kann dabei variieren, sodaß sowohl ein in Bezug auf die bei der Reaktion abgespaltene HCI-Menge molarer Unterschuß, eine äquimolare Menge aber auch ein molarer Überschuß an Trialkylamin verwendet werden kann. Bevorzugt wird jedoch ein molares Verhältnis von DHP zu Trialkylamin von etwa 1 : 2 bis 1 : 3, besonders bevorzugt bis 1 : 2,25 eingesetzt. Nach beendeter Dosierung wird das so erhaltene Reaktionsgemisch gegebenenfalls auf eine Temperatur bis 100°C erwärmt und etwa 10 bis 300 Minuten, bevorzugt etwa 20 bis 90 Minuten bei dieser Temperatur belassen. Bevorzugt wird das Reaktionsgemisch bei einer Temperatur von 40 bis 95°C, besonders bevorzugt von 65 bis 90°C, belassen.

Zur Isolierung und Aufarbeitung von DCP wird überschüssiges POCl₃ abdestilliert. Bevorzugt wird unter Vakuum abdestilliert. Die Sumpftemperatur kann dabei in Abhängigkeit vom erreichten Vakuum variieren, wobei die Untergrenze durch die Rührbarkeit des Reaktionsgemisches beeinflußt wird. Vorteilhafterweise wird der verbleibende Rückstand gleichzeitig mit einem Neutralisierungsmittel, bevorzugt mit Natron- oder Kalilauge, besonders bevorzugt mit 20 bis 50 %iger Natronlauge, in Wasser mit einer Temperatur von etwa 30 bis 70°C eingetragen, wobei die Geschwindigkeit der Zugabe so variiert wird, daß ein möglichst konstanter pH-Wert zwischen 2 und 5, bevorzugt von etwa 2 bis 3, erreicht wird und die Temperatur nicht über 70°C steigt. Anschließend wird noch etwa 10 bis 60 Minuten bei einer Temperatur zwischen etwa 30 und 70°C, bevorzugt zwischen 40 und 50°C, gerührt und das Reaktionsgemisch auf eine Temperatur zwischen etwa 10 und 30°C abgekühlt, worauf DCP ausfällt. Die weitere Aufarbeitung kann sodann durch Abfiltrieren, Waschen mit Wasser und Trocknen bei etwa 40°C im Vakuum erfolgen.
Zur weiteren Reinigung kann DCP gewünschtenfalls noch durch Destillation gereinigt werden. DCP kann jedoch auch nach dem Waschen mit Wasser mit einem inerten und mit Wasser nicht oder kaum mischbaren Lösungsmittel , das zuvor auf etwa 50 bis 65°C erwärmt wird, extrahiert und nach Abtrennung der wäßrigen Phase aus der Lösungsmittelphase durch fraktionierte Vakuumdestillation gewonnen werden. Geeignete Lösungsmittel sind dabei bevorzugt Toluol, Xylole, Cyclohexan oder Ether, wie etwa Methyl-tert.butylether, Diisopropylether, oder Mischungen davon.
Eine weitere Möglichkeit ist, daß das ausgefallene DCP ohne vorhergehendes Abfiltrieren und Waschen direkt mit dem inerten Lösungsmittel aus der Reaktionslösung extrahiert und wiederum durch fraktionierte Vakuumdestillation erhalten wird.

Mit dem erfindungsgemäßen Verfahren wird DCP in gegenüber dem Stand der Technik in hoher Reinheit (bis zu 99,9%) und in Ausbeuten von über 80% erhalten.

### Beispiel 1:

In einem 500 ml Doppelmantelglasreaktor mit Rührwerk, Rückflußkühler und Thermostatheizung wurden 90 g (0,80 mol) 4,6-Dihydroxypyrimidin und 592,0 g (3,86 mol) Phosphoroxychlorid vorgelegt und innerhalb von 120 Minuten 167,7 g (1,65 mol) Triethylamin zudosiert. Nach Ende der Triethylamin-Dosierung wurde das Reaktionsgemisch auf 85 °C erwärmt und 30 Minuten bei dieser Temperatur belassen.

Danach wurde überschüssiges Phosphoroxychlorid im Vakuum abdestilliert. In ein weiteres Reaktionsgefäß wurden 500 ml Wasser, das auf 40°C vorgewärmt wurde, vorgelegt in das gleichzeitig die Reaktionslösung sowie 50 %ige Natronlauge zudosiert wurden. Die Eintragung der beiden Lösungen (d.h. des Reaktionsgemisches aus der Chlorierung sowie die Natronlauge) in das Wasser erfolgte in einer solchen Geschwindigkeit, daß der pH Wert möglichst konstant bei pH 2,5 gehalten wurde und die Temperatur nichtüber 53°C anstieg.

Nach Ende der Dosierung wurde noch 30 Minuten bei 45°C gerührt. Anschließend wurde das Reaktionsgemisch auf 15°C abgekühlt, das Produkt abfiltriert, 3 x mit je 200 ml Wasser gewaschen und im Vakuum ei 40°C getrocknet.
- Ausbeute:: 106,2 g 4,6-Dichlorpyrimidin (= 89,1 % d. Th.)
- Analysen:: Gehalt (HPLC): 98,6 % w/w.

Durch Extraktion der Mutterlauge mit Toluol konnten weitere 3,2 g (= 2,7 % d. Th.) 4,6-Dichlorpyrimidin erhalten werden.

### Beispiel 2:

Analog Beispiel 1 wurden 90 g DHP mit 592 g POCl₃ in Gegenwart von 167,7 g Triethylamin umgesetzt.
Die Aufarbeitung erfolgte bis zum Abkühlen des Reaktionsgemisches ebenfalls analog Beispiel 1.

Das ausgefallene Produkt wurde anschließend abfiltriert und nur 1x mit 200 ml Wasser gewaschen.
Das filterfeuchte Produkt wurde sodann mit 400 ml auf 60°C erwärmten Toluol von der Filternutsche gewaschen. Nach Abtrennung der wäßrigen Phase im Scheidetrichter wurde die toluolische Lösung von 4,6-Dichlorpyrimidin im Vakuum fraktioniert destilliert.
Ausbeute: 100,4 g DCP (= 84,3 % d. Th.)
Gehalt (GC)): 99,9 % w/w

## Patentansprüche

1. Verfahren zur Herstellung von reinem 4,6-Dichlorpyrimidin, durch Reaktion von 4,6-Dihydroxypyrimidin mit einem Überschuß an Phosphoroxychlorid und in Gegenwart eines Säurefängers und Katalysators, dadurch gekennzeichnet, daß ein Trialkylamin mit 2 bis 4 C-Atomen im Alkylteil als Säurefänger und Katalysator verwendet wird, und daß zur Isolierung von 4,6-Dichlorpyrimidin überschüssiges Phosphoroxychlorid abdestilliert und der Rückstand, sowie ein Neutralisierungsmittel in Wasser bei 30 bis 70°C eindosiert wird, sodaß ein pH-Wert zwischen 2 und 5 erreicht wird, worauf nach 10 bis 60 minütigem Rühren und anschließendem Abkühlen auf 10 bis 30°C 4,6-Dichlorpyrimidin ausfällt

2. Verfahren nach Anspruch 1, dadurch gekennzeichnet, daß als Trialkylamin Triethylamin verwendet wird.

3. Verfahren nach Anspruch 1, dadurch gekennzeichnet, daß 4,6-Dihydroxypyrimidin und Phosphoroxychlorid in einem molaren Verhältnis von 1 : 3 bis 1 : 8 eingesetzt werden.

4. Verfahren nach Anspruch 1, dadurch gekennzeichnet, daß das Trialkylamin und 4,6-Dihydroxypyrimidin in einem molaren Verhältnis von 2 : 1 bis 3 : 1 eingesetzt werden.

5. Verfahren nach Anspruch 1, dadurch gekennzeichnet, daß als Neutralisierungsmittel Natronlauge oder Kalilauge verwendet wird.

6. Verfahren nach Anspruch 1, dadurch gekennzeichnet, daß ein pH-Wert zwischen 2 und 3 eingestellt wird.

7. Verfahren nach Anspruch 1, dadurch gekennzeichnet, daß das ausgefallene 4,6-Dichlorpyrimidin abfiltriert, mit Wasser gewaschen und im Vakuum getrocknet wird.

8. Verfahren nach Anspruch 1, dadurch gekennzeichnet, daß das ausgefallene 4,6-Dichlorpyrimidin, gegebenenfalls nach Abfiltrieren und Waschen mit Wasser, mit einem inerten, mit Wasser unmischbaren oder kaum mischbaren Lösungsmittel extrahiert und im Vakuum fraktioniert destilliert wird.

9. Verfahren nach Anspruch 8, dadurch gekennzeichnet, daß als inertes, mit Wasser unmischbares oder kaum mischbares Lösungsmittel Toluol, Xylole, Ether oder Cyclohexan, oder Mischungen davon verwendet werden.

## Revendications

1. Procédé de préparation de 4,6-dichloropyrimidine pure par réaction de 4,6-dihydroxypyrimidine avec un excès d'oxychlorure de phosphore et en présence d'un capteur d'acide et d'un catalyseur, caractérisé en ce qu'on utilise une trialkylamine ayant de 2 à 4 atomes de carbone dans la partie alkyle, en tant que capteur d'acide et catalyseur, et en ce que, pour isoler la 4,6-dichloropyrimidine, on élimine l'oxychlorure de phosphore en excès par distillation et on introduit le résidu ainsi qu'un agent neutralisant de façon dosée dans de l'eau à une température de 30 à 70°C de façon à atteindre une valeur de pH comprise entre 2 et 5, et après 10 à 60 minutes d'agitation suivie d'un refroidissement à une température de 10 à 30°C, la 4,6-dichloropyrimidine précipite.

2. Procédé selon la revendication 1, caractérisé en ce qu'on utilise de la triéthylamine en tant que trialkylamine.

3. Procédé selon la revendication 1, caractérisé en ce qu'on utilise de la 4,6-dihydroxypyrimidine et de l'oxychlorure de phosphore selon un rapport molaire de 1 : 3 à 1 : 8.

4. Procédé selon la revendication 1, caractérisé en ce qu'on utilise la trialkylamine et la 4,6-dihydroxypyrimidine selon un rapport molaire de 2 : 1 à 3 : 1.

5. Procédé selon la revendication 1, caractérisé en ce qu'on utilise une lessive de soude ou une lessive de potasse en tant qu'agent neutralisant.

6. Procédé selon la revendication 1, caractérisé en ce qu'on ajuste le pH à une valeur comprise entre 2 et 3.

7. Procédé selon la revendication 1, caractérisé en ce qu'on sépare la 4,6-dichloropyrimidine précipitée par filtration, on la lave avec de l'eau et on la sèche sous vide.

8. Procédé selon la revendication 1, caractérisé en ce qu'on extrait la 4,6-dichloropyrimidine précipitée avec un solvant inerte non miscible ou peu miscible avec l'eau, le cas échéant après séparation par filtration et lavage à l'eau, et on la sépare par distillation fractionnée sous vide.

9. Procédé selon la revendication 8, caractérisé en ce qu'on utilise, en tant que solvant inerte non miscible ou peu miscible avec l'eau, du toluène, du xylène, un éther ou du cyclohexane ou des mélanges de ceux-ci.

## Claims

1. Process for the preparation of pure 4,6-dichloropyrimidine by reaction of 4,6-dihydroxypyrimidine with an excess of phosphorus oxychloride in the presence of an acid scavenger and catalyst, characterized in that a trialkylamine having 2 to 4 C atoms in the alkyl moiety is used as acid scavenger and catalyst, and in that, to isolate 4,6-dichloropyrimidine, excess phosphorus oxychloride is distilled off and the residue and a neutralizing agent are added to water at 30 to 70°C, so that a pH between 2 and 5 is achieved, whereupon, after stirring for 10 to 60 minutes and subsequent cooling to 10 to 30°C, 4,6-dichloropyrimidine precipitates out.

2. Process according to Claim 1, characterized in that the trialkylamine used is triethylamine.

3. Process according to Claim 1, characterized in that 4,6-dihydroxypyrimidine and phosphorus oxychloride are used in a molar ratio of 1 : 3 to 1 : 8.

4. Process according to Claim 1, characterized in that the trialkylamine and 4,6-dihydroxypyrimidine are used in a molar ratio of 2 : 1 to 3 : 1.

5. Process according to Claim 1, characterized in that the neutralizing agent used is sodium hydroxide solution or potassium hydroxide solution.

6. Process according to Claim 1, characterized in that a pH between 2 and 3 is established.

7. Process according to Claim 1, characterized in that the precipitated 4,6-dichloropyrimidine is filtered ) off, washed with water and dried in vacuo.

8. Process according to Claim 1, characterized in that the precipitated 4,6-dichloropyrimidine is, if appropriate after filtration and washing with water, extracted with an inert water-immiscible or virtually water-immiscible solvent and fractionally distilled in vacuo.

9. Process according to Claim 8, characterized in that the inert water-immiscible or virtually water-immiscible solvent used is toluene, xylenes, ethane or cyclohexane, or mixtures thereof.
